# EUROPÄISCHE PATENTSCHRIFT

(11) **EP 1 335 760 B1**
(45) Veröffentlichungstag und Bekanntmachung des Hinweises auf die Patenterteilung: **30.08.2006**
(21) Anmeldenummer: 01997319.7
(22) Anmeldetag: 21.11.2001
(51) Int. Cl.: A61L 31/06, A61L 31/16, C08G 18/08

(54) **RÖHRENFÖRMIGE GEFÄSSIMPLANTATE (STENTS) SOWIE VERFAHREN ZU DEREN HERSTELLUNG**
TUBULAR VASCULAR IMPLANTS (STENTS) AND METHODS FOR PRODUCING THE SAME
TUTEURS INTRAVASCULAIRES TUBULAIRES (STENTS) ET LEURS PROCEDES DE PRODUCTION

(30) Priorität: 21.11.2000 DE 10057817; 27.11.2000 US 252891 P
(43) Veröffentlichungstag der Anmeldung: 20.08.2003
(73) Patentinhaber: Schering Aktiengesellschaft, 13353 Berlin (DE)
(72) Erfinder: HILDEBRAND, Gesine, 10555 Berlin (DE); TACK, Johannes, 13595 Berlin (DE); KÄUFER, H., 40882 Mettmann (DE); WACHE, Hans-Martin, 10245 Berlin (DE); MÜLLER, Thomas, 12277 Berlin (DE); EWERT, Peter, 12169 Berlin (DE)
(86) Internationale Anmeldenummer: PCT/DE2001/004424
(87) Internationale Veröffentlichungsnummer: WO 2002/041929

(56) Entgegenhaltungen:
- EP-A- 0 970 711
- WO-A-95/26762
- DE-A- 19 638 570
- DE-A- 19 755 872

## Beschreibung

Die Erfindung betrifft den in den Patentansprüchen gekennzeichneten Gegenstand, d.h. neue röhrenförmige Gefäßimplantate (Stents) sowie Verfahren zu deren Herstellung.

Herz/Kreislauferkrankungen sind weitverbreitete Krankheiten in den Industrienationen. Sie stellen eine der häufigsten Todesursachen dar. In den allermeisten Fällen werden Herz/Kreislauferkrankungen durch die Atherosklerose hervorgerufen. Diese ist eine entzündliche, fibroproliferative Erkrankung, die für 50 % aller Todesfälle in den USA, Europa und Japan verantwortlich ist (Ross 1993, Nature 362: 801-809). Mit ihrer peripheren Ausprägung bedroht sie den Erhalt der Extremitäten, mit ihrer koronaren Manifestation besteht das Risiko des tödlichen Herzinfarkts und mit supraaortalem Befall droht der Schlaganfall.

Eine Behandlung der Atherosklerose erfolgt derzeit auf unterschiedlichen Wegen. So hat sich neben den konservativen Maßnahmen (z. B. die Senkung des Cholesterinspiegels im Blut) und der Bypass-Operation, auch die mechanische Dilatation (Angioplastie) sowie die intravasale Entfernung atheromatösen Gewebes (Atherektomie) verengter Segmente in peripheren Arterien und den Koronarien als Alternative im klinischen Alltag etabliert.

Wie nachfolgend ausgeführt, sind die genannten Methoden jedoch mit einer Vielzahl von Nachteilen behaftet.

So wird der Wert mechanisch rekanalisierender Verfahren akut durch Gefäßverschlüsse in Folge von Gefäßeinrissen und -dissektionen sowie akuten Thrombosen beeinträchtigt (Sigwart et al. 1987, N. Engl. J. Med. 316: 701-706). Der langfristige Erfolg wird durch das Wiederauftreten von Einengungen (Restenosen) gefährdet. So ergab die CAVEAT-Studie an 1012 Patienten, daß die Restenoserate sechs Monate nach Intervention bei der koronaren Atherektomie 50 % und bei der koronaren Angioplastie sogar 57 % betrug (Topol et al. 1993, N. Engl. J. Med. 329: 221-227). Weiterhin traten in dieser Studie in 7% der Atherektomie- und in 3% der Angioplastie-Patienten abrupte Gefäßverschlüsse auf. Nicolini und Pepine (1992, Endovascular Surgery 72: 919-940) berichten von einer Restenoserate zwischen 35 und 40% und einer akuten Verschlußrate von 4% nach angioplastischen Eingriffen.

Um diesen Komplikationen zu begegnen, wurden verschiedene Techniken entwickelt. Hierzu gehört die Implantation metallischer Endoprothesen (Stents), (Sigwart et al. 1987, N. Engl. J. Med. 316: 701-706; Strecker et al., 1990, Radiology 175: 97-102). Die Stentimplantation in großkalibrigen Arterien, z.B. bei Okklusionen in der Beckenachse hat bereits den Rang einer primär anzuwendenden Therapiemodalität erhalten. Der Einsatz von Stents in den Femoralarterien hat dagegen mit einer primären Offenheitsrate von 49 % und einer Reokklusionshäufigkeit von 43 % enttäuschende Ergebnisse gezeigt (Sapoval et al., 1992, Radiology 184: 833-839). Ebenfalls unbefriedigende Resultate hauptsächlich bedingt durch Restenose, wurden mit bisher verfügbaren Stents in den Koronararterien erzielt (Kavas et al. 1992, J. Am. Coll. Cardiol 20: 467-474).

Als Ursache für die nach mechanischen Eingriffen häufig auftretenden Restenosen wird angenommen, daß die Eingriffe eine Proliferation und Migration glatter Muskelzellen in der Gefäßwand induzieren. Diese führen zu einer neointimalen Hyperplasie und den beobachteten Restenosen in den behandelten Gefäßabschnitten (Cascells 1992, Circulation 86: 723-729, Hanke et al. 1990, Circ. Res. 67: 651-659, Ross 1993, Nature 362: 801-809).

In der europäischen Patentschrift EP 706 376 werden Metalldrahtendoprothesen beschrieben, welche mit einem Polymer beschichtet sind, das als Wirkstoff Taxol enthält und diesen Wirkstoff nach und nach in das umliegende Gewebe abgibt. Taxol hat eine anti-angiogene Wirkung, und es wird behauptet, daß die so beschichteten Stents geeignet wären, die Restenose zu verhindern. Es werden allerdings keine Ergebnisse klinischer Studien zur Stützung dieser Hypothese offenbart.

In der deutschen Patentschrift DE 198 12 160 C1 werden Formkörper aus wirkstoffhaltigen thermoplastischen Polyurethanen beschrieben, welche antibiotisch wirksame Stoffe in homogener Verteilung enthalten. Die beschriebenen Formkörper sind insbesondere zentralvenöse Katheter.

Im US-Patent 5,962,004 werden Implantate beschrieben, die aus einem auf Polyurethan basierendem Material mit einer Glasübergangstemperatur zwischen 20°C und 60°C bestehen. Die Implantate weisen ein Formgedächtnis auf.

Im US-Patent 5,716,410 wird ein temporärer Stent und seine Verwendung beschrieben. Der Stent besteht aus Polyurethan und weist ein Formgedächtnis auf. Das Polyurethan hat eine Glasübergangstemperatur zwischen 40°C und 80°C, wobei eine Glasübergangstemperatur von 45°C bevorzugt ist.

In der internationalen Patentanmeldung WO 99/42528 werden Polymere beschrieben, die ein Formgedächtnis aufweisen. Die Übergangstemperatur liegt zwischen -30°C und 270°C. Bevorzugte Polymere sind Polyurethane, und diese können benutzt werden, um z.B. Stents herzustellen.

Im US-Patent US 5,458,935 werden thermoplastische Polyurethane beschrieben, die eine Härte zwischen 60 und 70 Shore D aufweisen. Die Polymere werden zur Herstellung von Schläuchen verwendet.

In der deutschen Offenlegungsschrift DE 197 55 872 A1 werden bereits Kunststoffteile mit Formgedächtnis aus thermoplastischem Kunststoff beschrieben, welche als Gefäßimplantate geeignet sind. Die dort beschriebenen Implantate können aus jedem beliebigen thermoplastischen Kunststoff gefertigt werden.

Alle bisherigen pharmakologischen und mechanischen Interventionen bei Patienten haben bis heute die Restenose nicht verhindern können (Muller et al. 1992, J. Am. Coll. Cardiol. 19: 418-432). Es besteht daher weiter ein Bedarf an geeigneten Mitteln, die für die Prophylaxe der Restenose geeignet sind.

Aufgabe der vorliegenden Erfindung ist es, besonders geeignete röhrenförmige Gefäßimplantate zu entwickeln, welche mindestens einen Wirkstoff enthalten, der gegebenenfalls in hoher Konzentration und über einen längeren Zeitraum an das umliegende Gewebe abgegeben wird.

Diese Aufgabe wird durch solche röhrenförmigen Gefäßimplantate gelöst, welche ein thermoplastisches Polyurethan mit einer Shore-Härte von 73A bis 80D umfassen, ein Formgedächtnis und eine Rückstelltemperatur zwischen 35°C und 50°C aufweisen, und mindestens einen Wirkstoff enthalten.

Überraschend wurde gefunden, daß sich aliphatische, polycarbonatbasierte thermoplastische Polyurethane besonders gut für die Herstellung von Gefäßimplantaten mit Formgedächtnis für die Prophylaxe der Restenose eignen. Besonders geeignet sind sogenannte Carbothane der Firma Thermedics, Inc., welche in einer großen Bandbreite von Härtegraden, Farben und Röntgenkontrastmittelzugaben erhältlich sind. Alle diese Kunststoffe sind geeignet für den Einsatz als medizinisch reine Biomaterialien und haben den U.S.P. Klasse VI-Test, den MEM Elution-Test und andere relevante Tests bestanden, um ihre Biokompatibilität und Biostabilität zu zeigen. Die Carbothane weisen eine außerordentlich hohe hydrolytische und Oxidationsstabilität auf, welche auf eine ausgezeichnete Langzeitbiostabilität schließen lassen.

Weiter wurde überraschend gefunden, daß sich Materialtypen mit einer hohen Härte besonders gut für den genannten Zweck eignen. Geeignete Polyurethane aus der Klasse der Carbothane haben eine Shore-Härte zwischen 73A und 80D . In den nachfolgenden Beispielen werden Versuche mit Polyurethanen unterschiedlicher Härte detailliert beschrieben, wobei herausgefunden wurde, daß das Polymer PC-3572D mit den in Tabelle 1 beschriebenen mechanischen Eigenschaften für die Herstellung von Gefäßimplantaten zur Prophylaxe der Restenose am besten geeignet ist.

**Tabelle 1: Mechanische Eigenschaften von Carbothane PC-3572D**

| **Produkt** | **Reißspannung [N/mm**^{**2**}**]** | **Bruchdehnung [%]** | **Zugmodul [N/mm**^{**2**}**]** | **Biegemodul [N/mm**^{**2**}**]** | **MFI 8 [g/10 min]** | **Härteprüfung [Shore]** |
|---|---|---|---|---|---|---|
| **PC-3572D** | **57.8** | **360** | **22.712** | **625.6** | **4.8 bei 210 °C** | **71D (hart)** |

Die Herstellung der Gefäßimplantate folgt im wesentlichen den Verfahren, welche in der deutschen Offenlegungsschrift DE 197 55 872 A1 beschrieben sind. Dort wird bereits ausgeführt, daß die Kunststoffteile im wesentlichen mit Hilfe zweier Verfahren hergestellt werden können: einerseits mit Hilfe des Spritzgießverfahrens, andererseits durch Extrusion.

Beim Spritzgießverfahren wird das Kunststoffgranulat bzw. -pulver in den Einfalltrichter der Spritzgießmaschine gefüllt, der es einem horizontalen Hohlzylinder zuführt, in dem es durch eine meist von einem Elektromotor angetriebene Schnecke durch Drehen weiterbefördert wird. Der Hohlzylinder ist beheizt, so dass infolge der Erwärmung über eine Turbulenz und die Scherung des Kunststoffes eine Plastifizierung erfolgt. Vor dem Schneckenende sammelt sich der plastifizierte Kunststoff. Durch eine meist ölhydraulisch bewirkte Vorwärtsbewegung der Schnecke als Kolben wird er dann in kurzer Zeit mit hoher Kraft in die geschlossene Form des Werkzeuges eingespritzt. Während das Formteil im meist wassergekühlten Werkzeug abkühlt, fördert die Schnecke bei gleichzeitiger Plastifizierung den Kunststoff für das nächste Spritzgießteil in den Vorratsraum vor die Schneckenspitze.

Das Spritzgießverfahren ist für röhrenförmige Teile in der Werkzeugeinrichtung aufwendig, da der Hohlraum durch einen Kern realisiert wird und dieser nach der Schwindung durch das Abkühlen des Materials schwer zu entfernen ist. Der Aufbau des Verfahrens ist zwar aufwendig, allerdings ist im Spritzgießverfahren eine sehr genaue und zuverlässige Fertigung möglich. Die Verarbeitungstemperatur der Carbothane-Schmelze beträgt bei diesem Verfahren 160 bis 240°C, vorzugsweise 180 bis 200°C.

Ein Extruder weist eine Plastifiziereinheit ähnlich der einer Spritzgießmaschine auf. Diese arbeitet jedoch mit ihrer Schnecke im Zylinder nicht in einen Vorratsraum hinein, sondern direkt in das düsenförmig ausgebildete Werkzeug, in dem die Formung stattfindet. Für die Fertigung jeglicher Art von endlosen Profilen, Röhren und Schläuchen ist die Extrusion ein besonders gut geeignetes Mittel, da äußerst wirtschafdich und reproduzierbar auch größere Mengen verarbeitet werden können. Die Verarbeitungstemperaturen von Carbothane liegen hier bei 200 bis 260°C, vorzugsweise bei 220°C.

Weiter können die erfindungsgemäßen Kunststoffteile prinzipiell auch durch Tauchen oder Gießen hergestellt werden, da die verwendeten Polyurethane in einer Vielzahl von Lösungsmitteln löslich sind. Als Lösungsmittel eignen sich beispielsweise Chloroform, Cyclohexan, Cyclohexanon, Cyclopentanon, Dimethylacetamid, Dimethylformamid, Dimethylsulfoxid, Dioxan, Methylenchlorid, N-Methyl-Pyrrolidon, Tetrahydrofuran, Acetonitril, Aceton, Diethylether, Ethanol, Ethylacetat, Hexan, Isopropylalkohol, Methanol, Methyl-Ethyl-Keton, Toluol, Trichlorethan, Salzsäure, Natronlauge oder Kochsalzlösung. Das Tauchen und Gießen ist im Vergleich zu den oben beschriebenen Extrusions- und Spritzgießverfahren zwar etwas aufwendiger, hat aber den Vorteil, daß im Gegensatz zu den genannten Verfahren keine hohen Temperaturen notwendig sind. Diese Art der Fertigung kann bei einer geplanten Zugabe von thermolabilen medizinischen Wirkstoffen die einzig mögliche Verarbeitungsmöglichkeit sein.

Bei der Herstellung durch Tauchen wird der thermoplastische Kunststoff mit Hilfe eines geeigneten Lösungsmittels in eine physikalische Lösung überführt. Anschließend wird durch kurzzeitiges Eintauchen eines Kernkörpers eine dünne Kunststoffschicht auf den Kern aufgebracht. Durch die Anzahl der Wiederholungen dieses Vorgangs wird die Wandstärke des dadurch gebildeten Kunststoffkörpers festgelegt. Desweiteren können Wandstärke und Qualität des Kunststoffkörpers durch die Konzentration des Kunststoffes im Lösungsmittel beeinflußt werden.

Die Gefäßimplantate enthalten mindestens einen Wirkstoff, wobei unter Wirkstoff sowohl medizinische Wirkstoffe, welche an das umliegende Gewebe abgegeben werden, als auch röntgendichte Materialien (Röntgenkontrastmittel), wie z.B. Bariumsulfat, Zirkoniumoxid oder iodhaltige Verbindungen, zu verstehen sind. Beispielsweise können die Wirkstoffe bei der Polymerisation direkt dem Monomer zugegeben werden und dann im Kunststoffpulver oder -granulat homogen verteilt vorliegen oder bei der Verarbeitung der Polyurethanschmelze oder -lösung zum Gefäßimplantat in gewünschter Menge zugegeben werden. Die Wirkstoffe werden bevorzugt im Polymer gelöst oder dispergiert, wobei das Lösen des Wirkstoffes sowohl in der Schmelze als auch in der organischen Lösung des Polymers durchgeführt werden kann. So kann eine Wirkstoffbeimischung von bis zu 30 Gew. % Wirkstoff im Polyurethan erreicht werden. Die Verarbeitung erfolgt wie oben beschrieben durch Extrusion, Spritzguß, Tauchen oder Gießen, wobei im Extrusions- bzw. Sprizgußverfahren nur thermisch beständige Wirkstoffe verwendet werden können. Geeignet sind neben Röntgenkontrastmitteln vor allem Wirkstoffe, die antiproliferativ auf Endothel und/oder glatte Muskelzellen wirken, bzw. solche, die über eine Verhinderung der Thrombusanlagerung an die Gefäßwand den Beginn einer überschießenden Neointimabildung verhindern. Diese Wirkstoffe können in ihrer Wirkweise ganz unterschiedlich ansetzen und können von ganz unterschiedlichen Anwendungsgebieten (z.B. Kardiologie, Onkologie) bekannt sein.

Sind sowohl der Wirkstoff als auch das Polymer in demselben Lösungsmittel löslich, kann eine Lösung hergestellt werden, die beide Stoffe enthält. Der Wirkstoffgehalt ist bis zu einem Anteil von 30 Gew. % im Verhältnis zum Polymer frei einstellbar. Werden Lösungen unterschiedlicher Wirkstoffkonzentration verwendet, so ist abhängig vom gewünschten Freisetzungsprofil ein Formkörper zu erstellen, der in jeder seiner verschiedenen Schichten unterschiedliche Wirkstoffkonzentrationen oder auch unterschiedliche Wirkstoffe aufweist.

Beispiele für geeignete Wirkstoffgruppen sind unter anderem Wirkstoffe, die die Zellproliferation eindämmen (z.B. Zytostatika, antiangiogene Wirksubstanzen), Wirkstoffe, welche eine Entzündung am Applikationsort verhindern (z.B. Corticoide, NSAID), oder Wirkstoffe, die Thrombenbildung hemmen (z.B. Heparin, Hirudin), oder Angiogenese-Wachstumsfaktoren. Besonders geeignet sind die Wirkstoffe Angiostatin, Endostatin, Iloprost, Prostacyclin, Endoxan, Methothrexat, Heparin, Hirudin, Clopidogrel, Paclitaxel, Doxazosin, Thalidomid, Rapamycin, Trapidil, Acetylsalicylsäure, Dexamethason, Prednisolon, Triamcinolonacetonid sowie Epothilon.

Aternativ können diese Wirkstoffe ausschließlich auf die innere und/oder äußere Oberfläche der Gefäßimplantate aufgebracht werden oder während des Tauchverfahrens in verschiedenen Konzentrationen in einzelnen Schichten eingebracht werden, um das Freisetzungsprofil des Wirkstoffs zu modellieren. Auf diese Art und Weise werden Gefäßimplantate hergestellt, bei denen die Wirkstoffkonzentration über den Querschnitt des Implantates variiert. Es ist auch möglich, den Kunststoff als Halbzeug oder als fertig gereckten Stent an der Oberfläche mit Wirkstoff zu bekleben oder den Wirkstoff in den plastifizierten Oberflächenbereich einzudrücken. Letzteres kann auch durch Walzen oder Pressen erfolgen. Ebenfalls ist es möglich, verschiedene Wirkstoffe in einem Stent zu verarbeiten, so daß z.B. ein Wirkstoff an die Gefäßwand und ein anderer Wirkstoff in das Gefäßlumen abgegeben wird.

Somit ist es möglich, Gefäßimplantate herzustellen, die an ihrer inneren und/oder äußeren Oberfläche, in einem bestimmten Oberflächenbereich oder im gesamten Bereich medizinische Wirkstoffe aufweisen.

In besonders günstigen Fällen beeinflussen die Wirkstoffe die Härte, Struktur und Festigkeit des Wirtspolymers positiv. Durch diesen Synergieeffekt kann die Zugabe des Polycarbonats reduziert werden.

Die Zugabe von Röntgenkontrastmitteln als Wirkstoffe ist dann notwendig, wenn das thermoplastische Polyurethan im Röntgenbild von dem umliegenden Gewebe unterschieden werden soll. Als Röntgenkontrastmittel sind stark absorbierende Stoffe wie z.B. Bariumsulfat, Zirkoniumoxid oder iodhaltige Verbindungen geeignet. Werden Kontrastmittel in das Polymer eingearbeitet, so wird das Kontrastmittel nicht gelöst, sondern lediglich dispergiert, so daß eine homogene Verteilung im Kunststoff vorliegt. Da das Kontrastmittel nicht in Lösung geht, verbleibt es nach dem Einbringen in den Organismus in der Polymermatrix, wird nicht aus dem Polymer herausgelöst und deshalb auch nicht wie die anderen medizinischen Wirkstoffe freigesetzt. Die Kontrastnüttelkonzentration im Kunststoff bleibt daher konstant.

Einem durch Spritzguß, Extrusion, Tauchen oder Gießen hergestellten Kunststoffteil wird dann wie bereits in DE 197 55 872 A1 beschrieben ein Formgedächtnis durch Recken und Fixieren aufgeprägt. Dieser Herstellungsschritt kann als die eigentliche Stentfertigung bezeichnet werden, bei dem aus dem Rohling das eigentliche Produkt in seinem fertigen Applikationsdurchmesser entsteht.

Der Vorgang des Reckens ist beim erfindungsgemäßen Verfahren ein Ziehen eines Probekörpers in die Länge. Dafür wird der Rohling eines Probekörpers mit einer definierten Länge in einer Einspannvorrichtung fixiert. Diese Einspannvorrichtung verhindert durch gezielten Druck auf die Probenenden ein späteres Verrutschen der Probe.

Der Druck (und damit die Einspannkraft) kann zum Beispiel mittels eines Druckluftzylinders auf die Probe ausgeübt werden. Es werden jeweils die Probenenden eingespannt. Der verwendete Probekörper hat beispielsweise eine Ausgangslänge von 70 mm bei einem Außendurchmesser von ca. 4 mm. Jedes Ende wird z.B. auf einer Länge von 10 mm eingespannt, so dass zum Recken eine Ausgangsprobenlänge zwischen den Einspannungen von ca. 50 mm vorliegt.

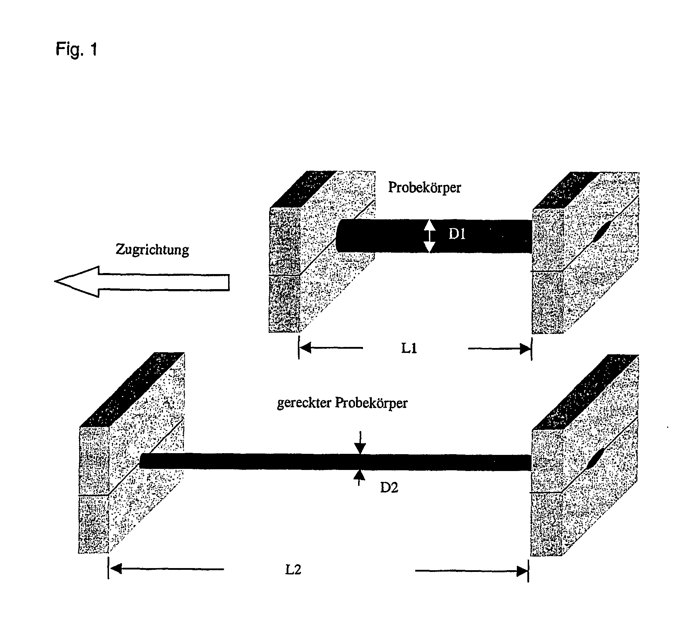

Um ein definiertes Temperaturprofil zu ermöglichen, befindet sich die Probe während des Reckvorganges in einem temperierten Fluid, vorzugsweise in einer temperierten Flüssigkeit wie z.B. Wasser. Fig. 1 zeigt schematisch einen Probekörper in seinen Ausgangsabmaßen, der in einem temperiertem Fluid in der Reckvorrichtung eingespannt ist.

Über einen Schrittmotor und eine Gewindestange können die Einspannungen in ihrem Abstand zueinander verändert werden. Werden die Einspannungen voneinander weg bewegt, so wird die eingespannte Probe in die Länge gezogen. Es ist darauf zu achten, daß die Haltekraft, die die Probeneinspannung auf die Probenenden ausübt, größer ist als die Zugkraft, die die Probe dem Zug entgegensetzt, da die Probe ansonsten aus der Einspannung herausrutschen würde.

Wie in Fig. 1 schematisch dargestellt, kann der Probekörper von einer Ausgangslänge L1 zwischen den Einspannungen (z.B. L1 = 50 mm) auf eine Endlänge L2 zwischen den Binspannungen (z.B. L2 = 150 mm) gereckt (gezogen) werden und erfährt dabei eine Durchmessereinschnürung von D1 (z.B. D1 = 4 mm) auf D2 (z.B. D2 = 2,5 mm).

Die Motordrehzahl kann variabel eingestellt werden, im vorliegenden Fall wurde eine Drehzahl von 20 U/min gewählt. Dies entspricht bei der vorliegenden Gewindesteigung der Spindel von 2,5mm einer Reckgeschwindigkeit von 50mm/min.

Im unteren Teil der Fig. 1 ist der Probekörper innerhalb des temperierten Fluids in gerecktem Zustand schematisch dargestellt.

Nach dem Reckvorgang baut der Probekörper seine inneren Spannungen durch die Relaxation des Kunststoffs weitgehend ab. Die Temperatur während der nun stattfindenden Fixierungsphase muß unbedingt konstant gehalten werden, denn diese Temperatur entspricht der späteren Aktivierungstemperatur. Nach der Fixierungsphase wird die zur Temperierung verwendete Flüssigkeit abgelassen und durch gekühlte Flüssigkeit ersetzt. Dadurch wird eine schnelle Abkühlung erreicht. Die Kühltemperatur wird so lange gehalten, bis der Probekörper durchgekühlt ist.

Zur Untersuchung der Rückstellung wird der Probekörper langsam mit einer Heizrate von 1°C/min erwärmt. Ab einer bestimmten Temperatur beginnt die Rückstellung der Probe. Mit Hilfe eines Meßwertaufnahmesystems können die Temperatur und der zurückgelegte Weg kontinuierlich aufgenommen werden. Innerhalb eines bestimmten Temperaturbereichs ist dabei ein Maximum der Rückstellung zu erwarten. Die Rückstellung endet, wenn die Probe wieder ihre Ausgangslänge von beispielsweise 70 mm erreicht hat. Ein Ausführungsbeispiel mit detaillierten Parametern ist in den nachfolgenden Beispielen beschrieben.

Das Rückstellmaximum ist so einzustellen, daß es zwischen 35°C und 50°C, beispielsweise bei 37°C, vorliegt. Diese Temperatur ist unabhängig von der Glasübergangstemperatur des Polymers. Sie wird ausschließlich von den beim Recken verwendeten Parametern bestimmt und ist in weiten Bereichen frei einstellbar. Der Temperaturbereich des Rückstellmaximums ist bei dem beschriebenen Verfahren eine ausschließlich verfahrenstechnisch und nicht werkstofftechnisch beeinflußbare Größe.

Der gereckte Stent kann dann mittels eines Katheters an den Applikationsort gebracht werden. Ein vorheriges Spülen des Katheters mit beispielsweise auf 10°C temperierte Kochsalzlösung kann eine Temperatur an der Katheterspitze von 31°C oder weniger für einen Zeitraum von 2 min nach der Spülung garantieren. Damit kann während der eigentlichen Stentapplikation ein definierter Temperatursprung erreicht werden, der die Applikation vereinfacht. Wird der Stent so appliziert, kann er formstabil an seinen Zielort gebracht werden und beginnt dort, ausgelöst durch die Körpertemperatur, mit der Rückstellung in seine Ausgangsform und damit mit der Aufweitung im Durchmesser. Die Durchmesseraufweitung spielt sich in einem Zeitraum von wenigen Minuten ab. Danach hat sich der Stent fest an die Gefäßwand gedrückt und kann nicht mehr verrutschen.

Die erfindungsgemäßen röhrenförmigen Gefäßimplantate eignen sich besonders gut für die Prophylaxe der Restenose, können allerdings - je nach Wirkstoffzusatz - auch als Gallengangstents oder in anderen Körperregionen implantiert werden. Die eingearbeiteten Arzneistoffe werden gemäß ihrer Löslichkeit und ihrem Verteilungskoeffizienten in das Blut bzw. in das umliegende Gewebe abgegeben. Zusätze, die lediglich zur Feststellung der Position mittels Röntgen dienen, z.B. das wasserunlösliche Bariumsulfat, werden nicht freigesetzt. So kann die Position des Stents auch nach der Implantation immer wieder bestimmt werden.

Die nachfolgenden Beispiele verdeutlichen die Erfindung, ohne sie auf diese beschränken zu wollen.

### Beispiel 1: Vergleich von Kunststoffteilen, welche aus Polyurethanen verschiedener Härtegrade gefertigt wurden

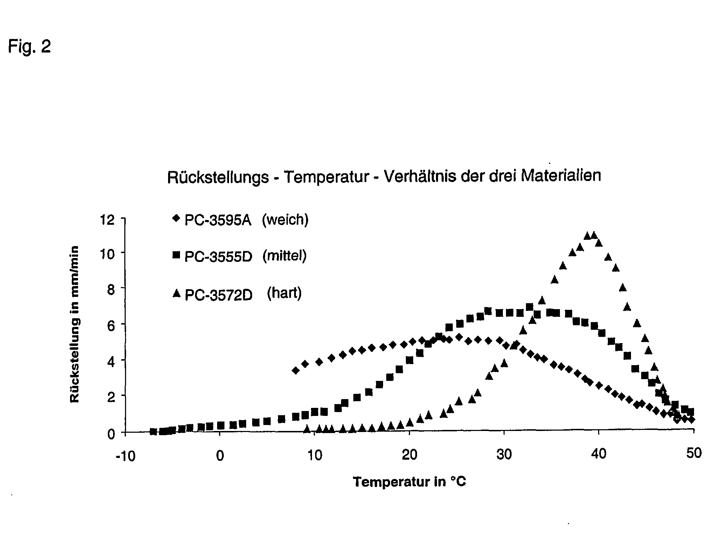

Es wurden Versuche zur Probenelastizität mit den Carbothanen PC 3595A (weich), PC 3555D (mittel) und PC 3572D (hart) durchgeführt. Diese drei Polyurethahtypen unterscheiden sich hinsichtlich ihrer Elastizität und Festigkeit. Das Rückstellverhalten dieser drei Typen ist in Fig. 2 dargestellt.

Dieser direkte Vergleich zeigt deutlich, daß die Rückstellkurven der weicheren Typen der Probekörper deutlich weitere Rückstelltemperaturbereiche aufweisen. Die weiche Variante der Proben läßt ein Rückstellmaximum kaum noch erkennen. Bei diesen Proben wird die Rückstellung der eingebrachten Orientierungen von reiner elastischer Rückstellung überlagert. Für den geplanten Einsatz als Kunststoffstent ist also der harte Polyurethantyp PC-3572D am besten geeignet, da er die am deutlichsten ausgeprägte Rückstellung hat. Desweiteren läßt das harte Material aufgrund der hohen Festigkeit auch sehr geringe Wanddicken bei hoher Stabilität zu, was zusätzlich für die Verwendung dieses Typen spricht.

### Beispiel 2: Herstellung von Kunststoffteilen mittels Extrusion

Die Extrusion der Carbothane erfolgte in einem Extruder anhand folgender Eckdaten:

**Tabelle 2: Extrusionsparameter von Carbothane**

| Produkt | Zone 1 [°C] | Zone 2 [°C] | Zone 3 [°C] | Zone 4 [°C] | Schmelze [°C] | Düse [°C] | Druck [N/mm²] |
|---|---|---|---|---|---|---|---|
| PC-3572D | 200 | 205 | 215 | 225 | 220 | 220 | 13.5-27 |

Die Extrusion erfolgte wie oben beschrieben. Der Außendurchmesser der Probekörper betrug 4 mm bei einer Wanddicke von 0,5 mm.

### Beispiel 3: Herstellung von Kunststoffteilen durch Spritzguß

Der Spritzguß wurde anhand des folgenden Temperaturprofils durchgeführt:

| Produkt | Einzugstemperatur [°C] | Massentemperatur [°C] | Düsentemperatur [°C] |
|---|---|---|---|
| PC3572D | 190 | 200 | 205 |

Das Spritzgießen erfolgte wie oben beschrieben. Die Abmaße der Probekörper entsprachen denen der Extrusion, wobei für den Kern eine Entformungsschräge von 1-2 % vorgesehen wurde.

### Beispiel 4: Herstellung von Kunststoffteilen durch Tauchen

Es wurde eine 5 %ige Carbothanlösung in Tetrahydrofuran hergestellt. Ein Tauchstab mit einem Außendurchmesser von 3 mm wurde für 15 Sekunden in die Lösung getaucht. Der benetzte Stab wurde aus der Lösung gehoben und in einem Wärmestrom für 30 Minuten unter Drehen getrocknet. Der Tauchvorgang wurde mehrere Male wiederholt bis die gewünschte Wanddicke erreicht war. Das fertige Polymerteil wurde von dem Metallstab abgezogen.
Das Tauchverfahren kann prinzipiell auch mit anderen Lösungsmitteln wie z.B. Chloroform durchgeführt werden.

### Beispiel 5: Recken, Fixieren und Messen der Rückstellung

Als eigentliche Stentfertigung ist das Einbringen des Formgedächtnisses, also das Recken mit dem anschließenden Fixieren, zu bezeichnen. In diesem Verfahrensschritt wird aus dem Rohling das eigentliche Produkt in seinem Applikationsdurchmesser. Desweiteren wird in diesem Verfahrensschritt durch Festlegung der Reckparameter die gewünschte Aktivierungstemperatur bestimmt.

Dafür wurde die vorbereitete Probe so in die Einspannvorrichtung eingespannt, daß sie zwischen den Einspannungen eine Länge von 50 mm hatte. Die Probe wurde mit Hilfe des Temperierkreislaufs auf 40°C erwärmt. Diese Temperatur wurde 10 min gehalten, damit die Probe durchgewärmt war. Die Probe wurde auf 200 % gedehnt. Der Motor arbeitete mit einer Drehzahl von 20 min-1 (das entspricht bei einer Gewindesteigung der Spindel von 2,5 mm einer Reckung von 50 mm/min). Nach einer Minute hatte sich die Probe auf 100 mm zwischen den Einspannungen gedehnt, der Motor schaltete ab. Anschließend baute der Probekörper seine inneren Spannungen durch die Relaxation des Kunststoffes weitestgehend ab. Die Temperatur während dieser sogenannten Fixierung wurde konstant gehalten, denn diese Fixiertemperatur entspricht der späteren Aktivierungstemperatur. Nach 15 Minuten wurde die temperierte Flüssigkeit abgelassen und durch gekühlte Flüssigkeit ersetzt. Diese Temperatur wurde einige Minuten gehalten, damit der Probekörper durchkühlen konnte. Die auch im erkalteten Zustand vorhandene Restspannung in der Probe baute sich nun durch ein zügiges Nachfahren des Schlittens ab. Bei der anliegenden Temperatur war der Zustand der Probe stabil.

### Untersuchung des Formgedächtnisverhaltens (Rückstellung):

Für die Untersuchung der Rückstellung wurde die Probe langsam erwärmt. Es wurde eine Heizrate von 1°C/min gewählt. Ab einer bestimmten Temperatur begann die Rückstellung der Probe. Das Meßwertaufnahmesystem nahm dabei kontinuierlich die Temperatur und den zurückgelegten Weg auf. Innerhalb eines bestimmten Temperaturbereiches wurde ein Maximum der Rückstellung erwartet. Die Rückstellung endete, wenn die Probe wieder nahezu ihre Ausgangslänge von 50 mm erreicht hatte. Die aufgenommenen Rohdaten standen nun zu einer weiteren Auswertung zur Verfügung.

### Parameter der Versuchsdurchführung:

**Tabelle 3: Standardparameter**

| Parameter | 11 Wert | Erläuterung |
|---|---|---|
| Recktemperatur | 40° C | Diese Temperatur muß mindestens so hoch sein wie die Fixiertemperatur. |
| Reckgeschwindigkeit | 50 mm/min | Bei dieser Geschwindigkeit werden die Moleküle gleichmäßig gereckt, und die dabei entstehende Wärme kann abgeführt werden. |
| Reckungsgrad | 100-400% | Je nach Reißdehnung des Materials sind verschiedene Reckungsgrade möglich. |
| Fixiertemperatur | 40°C | Bei dieser Temperatur wird das Rückstellmaximum erwartet. |
| Fixierzeit | 15 min | In dieser Zeit hat der Werkstoff nahezu alle Eigenspannungen abgebaut, wenn konstante Temperatur erreicht wird. |
| Einfriergeschwingigkeit | min. 20°C/min | Es ist eine schnelle Abkühlung anzustreben. |
| Einfriertemperatur | -10°C | Bei dieser Temperatur werden die Verformungen "eingefroren" |
| Heizrate | 1°C / min | Um gezielt die Rückstellung über die Temperatur aufnehmen zu können, ist ein langsames, gleichmäßiges Aufheizen erforderlich. |

### Beispiel 6: Wirkstoffbeladene Kunststoffteile

### a) Herstellung durch Extrusion

Dem Granulat von PC 3572D wurde 2% Dexamethason beigemischt. Die Extrusion erfolgte mit den in Beispiel 2 beschriebenen Parametern. Analog wurden Stents mit den Wirkstoffen Prednisolon und Triamcinolonacetonid in Konzentrationen von 0,5 bis 10 % des Wirkstoffs hergestellt.

### b) Herstellung durch Spritzguß

Dem Granulat von PC 3572D wurde 2% Dexamethason beigemischt. Das Spritzgießverfahren erfolgte mit den in Beispiel 3 beschriebenen Parametern. Analog wurden Stents mit den Wirkstoffen Prednisolon und Triamcinolonacetonid in Konzentrationen von 0,5 bis 10% des Wirkstoffs hergestellt.

### c) Herstellung durch Tauchen

Die Herstellung erfolgte wie in Beispiel 4, aber mit einer Beimischung von 2 % Dexamethason zu der Polymerlösung. Analog wurden Stents mit den Wirkstoffen Prednisolon, Acetylsalicylsäure, Iloprost und Triamcinolonacetonid in Konzentrationen von 0,5 bis 10% des Wirkstoffs hergestellt.

### d) Herstellung durch Tauchen in Lösungen mit unterschiedlicher Wirkstoffkonzentration

Die Herstellung erfolgte wie in Beispiel 4, aber mit Beimischungen von Dexamethason in Konzentrationen von 0,5 bis 10% in den unterschiedlichen Schichten, so daß der Probekörper zur Innenseite die höchste Konzentration und an der Außenseite die geringste Wirkstoffkonzentration aufwies.

### e) Herstellung duch Tauchen in Lösungen mit unterschiedlichen Wirkstoffzugaben

Die Herstellung erfolgte wie in Beispiel 4, aber mit Beimischungen von 2 % Dexamethason in der Lösung, aus der die innenliegenden Schichten getaucht wurden, und 2 % Iloprost, aus der die außenliegenden Schichten getaucht wurden. Zwischen den Schichten mit unterschiedlicher Wirkstoftbeladung wurde eine Trennschicht aus wirkstofffreiem Polyurethan getaucht.

### f) Freisetzungsstudien

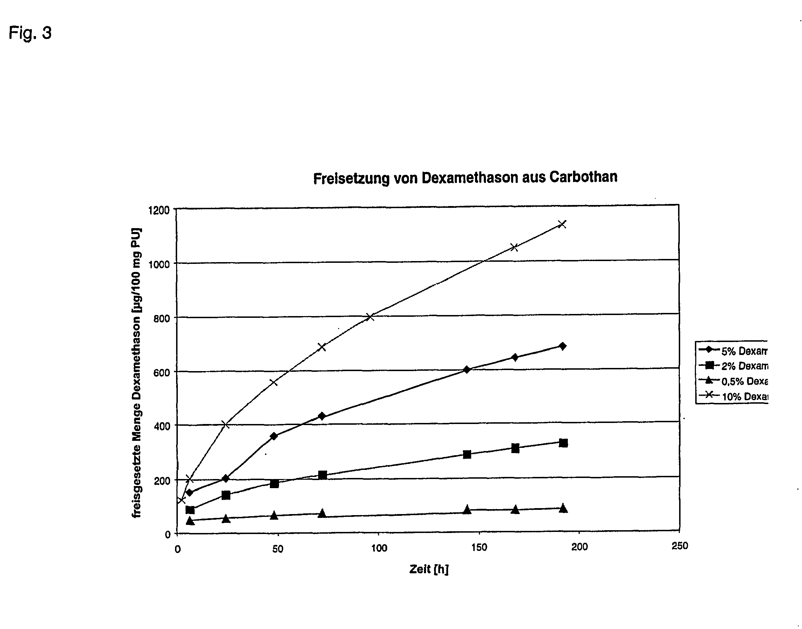

Die Freisetzung des Dexamethasons bei den nach Beispiel 6 c) hergestellten Stents wurde über einen Zeitraum von 7 Tagen in vitro bestimmt. Es wurde eine kontinuierliche Freisetzung des Wirkstoffes über den Beobachtungszeitrauni festgestellt (siehe Fig. 3).

Die Freisetzungsstudien bei den mit den anderen medizinischen Wirkstoffen beladenen Stents nahmen einen ähnlichen Verlauf.

### g) Recken und Fixieren der wirkstoffbeladenen Kunststoffteile

Das Recken und Fixieren der mit Wirkstoffen beladenen Kunststoffteile (Stents) erfolgte analog zu dem oben in Beispiel 5 beschriebenen Verfahren. Durch diese Behandlung wurde den Stents ein Formgedächtnis aufgeprägt.

### h) Herstellung von Kunststoffteilen mit Zusatz von Kontrastmittel

Im Spritzgußverfahren wurden Prüfstäbe mit einem Anteil von 10% Bariumsulfat gefertigt und mit Prüfstäben ohne Kontrastmittelzusatz verglichen. Es zeigte sich, daß das Material durch Zugabe von Bariumsulfat steifer wird: die Streckspannung nahm um ca. 20% zu, die Streckdehnung nahm um ca. 20% ab, das Elastizitätsmodul nahm um ca. 100% zu. Die Veränderungen der mechanischen Eigenschaften wirkten sich allerdings nur relativ gering auf das Rückstellverhalten aus: Innerhalb der ersten 5 Minuten der Rückstellung war keine signifikante Änderung der Rückstellung zu beobachten, danach vollzog sich die Rückstellung ein wenig langsamer. Die Rückstellung des mit Kontrastmittel beladenen Probekörpers erreichte ca. 89 % der Rückstellung des kontrastmittelfreien Probekörpers.

## Patentansprüche

1. Röhrenförmiges Gefäßimplantat (Stent), umfassend ein thermoplastisches Polyurethan mit einer Shore-Härte von 73A bis 80D, wobei das Gefäßimplantat ein Formgedächtnis und eine Rückstelltemperatur zwischen 35°C und 50°C aufweist und mindestens einen Wirkstoff enthält.

2. Röhrenförmiges Gefäßimplantat gemäß Anspruch 1, **dadurch gekennzeichnet, daß** ausschließlich die innere und/oder äußere Oberfläche des Gefäßimplantats einen Wirkstoff enthält.

3. Röhrenförmiges Gefäßimplantat gemäß Anspruch 1, **dadurch gekennzeichnet, daß** die Wirkstoffkonzentration über den Querschnitt des Implantates variiert.

4. Röhrenförmiges Gefäßimplantat gemäß einem der Ansprüche 1 bis 3, **dadurch gekennzeichnet, daß** das Gefäßimplantat zwei oder mehrere verschiedene Wirkstoffe enthält.

5. Röhrenförmiges Gefäßimplantat gemäß Anspruch 1 oder 4, **dadurch gekennzeichnet, daß** der Wirkstoff ein Röntgenkontrastmittel ist.

6. Röhrenförmiges Gefäßimplantat gemäß Anspruch 5, **dadurch gekennzeichnet, daß** das Röntgenkontrastmittel Bariumsulfat ist.

7. Röhrenförmiges Gefäßimplantat gemäß einem der vorherigen Ansprüche, **dadurch gekennzeichnet, daß** der Wirkstoff bis zu einem Anteil von 30 Gew.% im Verhältnis zum Polymer vorliegt.

8. Röhrenförmiges Gefäßimplantat gemäß einem der vorherigen Ansprüche, **dadurch gekennzeichnet, daß** der Wirkstoff nach der Implantation kontinuierlich freigesetzt wird.

9. Verfahren zur Herstellung von Gefäßimplantaten gemäß einem der Ansprüche 1 bis 8, **dadurch gekennzeichnet, daß** ein thermoplastisches Polyurethan zusammen mit einem Wirkstoff im Spritzgießverfahren bei 160 bis 240°C zu einem Rohling verarbeitet wird, und diesem Rohling durch Recken und Fixieren ein Formgedächtnis aufgeprägt wird.

10. Verfahren zur Herstellung von Gefäßimplantaten gemäß einem der Ansprüche 1 bis 8, **dadurch gekennzeichnet, daß** ein thermoplastisches Polyurethan zusammen mit einem Wirkstoff bei Temperaturen von 200 bis 260°C zu einem Rohling extrudiert wird, und diesem Rohling durch Recken und Fixieren ein Formgedächtnis aufgeprägt wird.

11. Verfahren zur Herstellung von Gefäßimplantaten gemäß einem der Ansprüche 1 bis 8, **dadurch gekennzeichnet, daß** ein thermoplastisches Polyurethan zusammen mit einem Wirkstoff in einem geeigneten Lösungsmittel gelöst wird, ein Rohling durch Tauchen oder Gießen der Lösung hergestellt wird, und diesem Rohling durch Recken und Fixieren ein Formgedächtnis aufgeprägt wird.

## Claims

1. Tubular vascular implant (stent) that comprises a thermoplastic polyurethane with a Shore hardness of 73A to 80D, which has a shape memory and a shape-recovery temperature of between 35°C and 50°C and contains at least one active ingredient.

2. Tubular vascular implant according to Claim 1, **characterized in that** only the inside surface and/or the outside surface of the vascular implant contains an active ingredient.

3. Tubular vascular implant according to Claim 1, **characterized in that** the active ingredient concentration varies via the cross-section of the implant.

4. Tubular vascular implant according to one of Claims 1 to 3, **characterized in that** the vascular implant contains two or more different active ingredients.

5. Tubular vascular implant according to Claim 1 or 4, **characterized in that** the active ingredient is an x-ray contrast medium.

6. Tubular vascular implant according to Claim 5, **characterized in that** the x-ray contrast medium is barium sulphate.

7. Tubular vascular implant according to one of the preceding claims, **characterized in that** the active ingredient is present up to a proportion of 30% by weight in relation to the polymer.

8. Tubular vascular implant according to one of the preceding claims, **characterized in that** the active ingredient is released continuously after the implantation.

9. Process for the production of vascular implants according to one of Claims 1 to 8, **characterized in that** a thermoplastic polyurethane together with an active ingredient is processed into a blank in the injection-moulding process at 160 to 240°C, and a shape memory is impressed on this blank by stretching and setting.

10. Process for the production of vascular implants according to one of Claims 1 to 8, **characterized in that** a thermoplastic polyurethane together with an active ingredient is extruded at temperatures of 200 to 260°C in a blank, and a shape memory is impressed on this blank by stretching and setting.

11. Process for the production of vascular implants according to one of Claims 1 to 8, **characterized in that** a thermoplastic polyurethane together with an active ingredient is dissolved in a suitable solvent, a blank is produced by immersion or pouring of the solution, and a shape memory is impressed on this blank by stretching and setting.

## Revendications

1. Implant vasculaire tubulaire (stent), comprenant un polyuréthanne thermoplastique ayant une dureté Shore de 73A à 80D, l'implant vasculaire présentant une mémoire de forme et une température de rappel comprise entre 35 °C et 50 °C et contenant au moins une substance active.

2. Implant vasculaire tubulaire selon la revendication 1, **caractérisé en ce qu'**exclusivement la surface interne et/ou la surface externe de l'implant vasculaire contient une substance active.

3. Implant vasculaire tubulaire selon la revendication 1, **caractérisé en ce que** la concentration de substance active varie sur la section transversale de l'implant.

4. Implant vasculaire tubulaire selon l'une quelconque des revendications 1 à 3, **caractérisé en ce que** l'implant vasculaire contient deux ou plus de deux substances actives différentes.

5. Implant vasculaire tubulaire selon la revendication 1 ou 4, **caractérisé en ce que** la substance active est un agent de contraste radiographique.

6. Implant vasculaire tubulaire selon la revendication 5, **caractérisé en ce que** l'agent de contraste radiographique est le sulfate de baryum.

7. Implant vasculaire tubulaire selon l'une quelconque des revendications précédentes, **caractérisé en ce que** la substance active est présente jusqu'à une proportion de 30 % en poids par rapport au polymère.

8. Implant vasculaire tubulaire selon l'une quelconque des revendications précédentes, **caractérisé en ce que** la substance active est libérée en continu après l'implantation.

9. Procédé pour la fabrication d'implants vasculaires selon l'une quelconque des revendications 1 à 8, **caractérisé en ce que** dans le procédé de moulage par injection à 160-240 °C on transforme en une ébauche un polyuréthanne thermoplastique conjointement avec une substance active, et on imprime à cette ébauche une mémoire de forme par étirage et fixage.

10. Procédé pour la fabrication d'implants vasculaires selon l'une quelconque des revendications 1 à 8, **caractérisé en ce qu'**un polyuréthanne thermoplastique est extrudé conjointement avec une substance active à des températures de 200 à 260°C en une ébauche, et on imprime à cette ébauche une mémoire de forme par étirage et fixage.

11. Procédé pour la fabrication d'implants vasculaires selon l'une quelconque des revendications 1 à 8, **caractérisé en ce qu'**on dissout dans un solvant approprié un polyuréthanne thermoplastique conjointement avec une substance active, on produit une ébauche par trempage ou coulée de la solution, et on imprime à cette ébauche une mémoire de forme par étirage et fixage.
